# EUROPEAN PATENT APPLICATION

(11) **EP 3 741 223 A1**
(43) Date of publication of application: **25.11.2020**
(21) Application number: 19741624.1
(22) Date of filing: 17.01.2019
(51) Int. Cl.: A23L 33/14, A61K 31/736, A61K 36/064, A61P 1/00, A61P 3/04, C12N 15/09, C12Q 1/686

(54) **INTESTINAL FB RATIO REDUCING AGENT AND FOOD COMPOSITION CONTAINING SAME**

(30) Priority: 18.01.2018 JP 2018006220
(71) Applicant: Asahi Group Holdings, Ltd., Tokyo 130-8602 (JP)
(72) Inventor: TANIHIRO Reiko, Moriya-shi, Ibaraki 302-0106 (JP)
(74) Representative: Hoffmann Eitle
(86) International application number: PCT/JP2019/001179
(87) International publication number: WO 2019/142844

(57) **Abstract**

The present invention provides: an agent having an FB ratio reducing effect in the intestines (intestinal FB ratio reducing agent) and being suitable for use in food/beverage products; and a food product composition for FB ratio reduction containing the agent. The intestinal FB ratio reducing agent of the present invention is one containing yeast mannan, and the food product composition of the present invention for FB ratio reduction is one containing the intestinal FB ratio reducing agent. The term "FB ratio" herein may be defined as [(occupancy of bacteria belonging to phylum Firmicutes)/(occupancy of bacteria belonging to phylum Bacteroidetes)], or as [(total occupancy of bacteria belonging to class Clostridia and bacteria belonging to class Erysipelotrichia among bacteria belonging to phylum Firmicutes)/(occupancy of bacteria belonging to class Bacteroidia among bacteria belonging to phylum Bacteroidetes)].

SEQUENCE LISTING

<110>  Asahi Group Holdings, Ltd.

<120>  Agent for decreasing enteric FB ratio and food composition
       comprising thereof

<130>  PA52-9004WO

<150>  JP 2018-6220
<151>  2018-1-18

<160>  8

<170>  PatentIn version 3.5

<210>  1
<211>  52
<212>  DNA
<213>  Artificial Sequence

<220>
<223>  Forward Primer for 16S rDNA

<400>  1
tcgtcggcag cgtcagatgt gtataagaga caggtgccag cmgccgcggt aa                52

<210>  2
<211>  55
<212>  DNA
<213>  Artificial Sequence

<220>
<223>  Reverse Primer for 16S rDNA

<400>  2
gtctcgtggg ctcggagatg tgtataagag acaggactac hvgggtatct aatcc             55

<210>  3
<211>  21
<212>  DNA
<213>  Artificial Sequence

<220>
<223>  Forward primer specific for bacteria of phylum Firmicutes

<400>  3
tgaaactyaa aggaattgac g                                                  21

<210>  4
<211>  17
<212>  DNA
<213>  Artificial Sequence

<220>
<223>  Reverse primer specific for bacteria of phylum Firmicutes

<400>  3
accatgcacc acctgtc                                                       17

<210>  5
<211>  20
<212>  DNA
<213>  Artificial Sequence

<220>
<223>  Forward primer specific for bacteria of phylum Bacteroidetes

<400>  5
craacaggat tagataccct                                                   20


<210>  6
<211>  19
<212>  DNA
<213>  Artificial Sequence

<220>
<223>  Reverse primer specific for bacteria of phylum Bacteroidetes

<400>  6
ggtaaggttc ctcgcgtat                                                    19

<210>  7
<211>  18
<212>  DNA
<213>  Artificial Sequence

<220>
<223>  Forward primer specific for eubacteria

<400>  7
cggtgaatac gttcccgg                                                     18


<210>  8
<211>  22
<212>  DNA
<213>  Artificial Sequence

<220>
<223>  Reverse primer specific for bacteria of phylum Bacteroidetes

<400>  8
tacggctacc ttgttacgac tt                                                22

SEQUENCE LISTING

<110>  Asahi Group Holdings, Ltd.

<120>  Agent for decreasing enteric FB ratio and food composition
       comprising thereof

<130>  PA52-9004WO

<150>  JP 2018-6220
<151>  2018-1-18

<160>  8

<170>  PatentIn version 3.5

<210>  1
<211>  52
<212>  DNA
<213>  Artificial Sequence

<220>
<223>  Forward Primer for 16S rDNA

<400>  1
tcgtcggcag cgtcagatgt gtataagaga caggtgccag cmgccgcggt aa                52

<210>  2
<211>  55
<212>  DNA
<213>  Artificial Sequence

<220>
<223>  Reverse Primer for 16S rDNA

<400>  2
gtctcgtggg ctcggagatg tgtataagag acaggactac hvgggtatct aatcc             55

<210>  3
<211>  21
<212>  DNA
<213>  Artificial Sequence

<220>
<223>  Forward primer specific for bacteria of phylum Firmicutes

<400>  3
tgaaactyaa aggaattgac g                                                  21

<210>  4
<211>  17
<212>  DNA
<213>  Artificial Sequence

<220>
<223>  Reverse primer specific for bacteria of phylum Firmicutes

<400>  3
accatgcacc acctgtc                                                       17

<210>  5
<211>  20
<212>  DNA
<213>  Artificial Sequence

<220>
<223>  Forward primer specific for bacteria of phylum Bacteroidetes

<400>  5
craacaggat tagataccct                                                   20


<210>  6
<211>  19
<212>  DNA
<213>  Artificial Sequence

<220>
<223>  Reverse primer specific for bacteria of phylum Bacteroidetes

<400>  6
ggtaaggttc ctcgcgtat                                                    19

<210>  7
<211>  18
<212>  DNA
<213>  Artificial Sequence

<220>
<223>  Forward primer specific for eubacteria

<400>  7
cggtgaatac gttcccgg                                                     18


<210>  8
<211>  22
<212>  DNA
<213>  Artificial Sequence

<220>
<223>  Reverse primer specific for bacteria of phylum Bacteroidetes

<400>  8
tacggctacc ttgttacgac tt                                                22

## Description

### Technical Field

The present invention relates to a prebiotic that promotes proliferation and activation of intestinal microflora, in particular, bacteria belonging to the phylum Bacteroidetes. The present invention also relates to uses of mannan extracted from yeast cell walls (yeast mannan).

### Background Art

A wide variety of bacteria are living in the intestines of mammals such as humans and mice to form an intestinal microflora, and one example of them is bacteria belonging to the phylum Bacteroidetes. Examples of dominant species of bacteria belonging to the phylum Bacteroidetes include Bacteroides thetaiotaomicron (B. thetaiotaomicron) and Bacteroides ovatus (B. ovatus). B. thetaiotaomicron is known to be one of seven bacterial species characteristically abundant in the intestines of lean individuals. It has been recently known that bacteria belonging to the phylum Bacteroidetes are abundant in the intestines of lean individuals and bacteria belonging to the phylum Firmicutes are abundant in the intestines of obese individuals, and FB ratio = (occupancy of phylum Firmicutes)/(occupancy of phylum Bacteoirdetes) is increasingly used as one of indexes of tendency of obesity (Non Patent Literature 1).

Patent Literature 1 (JP 2010-254594 A) describes an agent for accelerating proliferation of intestinal Bacteroides, the agent containing at least one of isomaltooligosaccharide, galactooligosaccharide, mannan oligosaccharide, a dietary fiber (e.g., indigestible dextrin), and a sugar absorption inhibitor (e.g., extract from a plant belonging to the genus Salacia), and also discloses, for example, that ingestion of the agent for accelerating proliferation of intestinal Bacteroides promotes the growth of intestinal Bacteroides, which is expected, for example, to enhance immune functions. Examples, in which agents for accelerating proliferation of intestinal Bacteroides with different components (e.g., an agent containing indigestible dextrin; an agent containing mannan oligosaccharide is not used) are used, demonstrate how the fraction (the percentage as the total number of bacterial cells is assumed as 100%) of Bacteroides in the intestinal microflora after ingestion changed from that before ingestion.

Cell walls of yeasts used for production of beer, sake, bread, and so on are rich in dietary fibers including glucan and mannan. Mannan purified from yeast cell walls is known as "yeast mannan", and researched for application as a functional material having various operative advantageous effects including actions relating to intestinal regulation, iron-absorption-promoting effect, antiulcer action, and diabetes-ameliorating effect.

### Citation List

### Patent Literature

Patent Literature 1: JP 2010-254594 A

### Non Patent Literature

Non Patent Literature 1: Ley, RE., Turnbaugh, PJ., Klein, S., Gordon, JI., Nature, 2006, 53, 65-69

### Summary of Invention

### Technical Problem

Foods with health claims (e.g., foods with functional claims) that are capable of improving the FB ratio of intestinal bacteria, that is, reducing the FB ratio to a value for lean individuals, or an agent that serves as an active ingredient (e.g., a functional substance) of them are desired, whereas those having sufficient improving effect have not yet been developed.

While "dietary fibers" are exemplified in Patent Literature 1, uses of insoluble dietary fibers thereamong are restricted to particular food forms because of the insolubility; on the other hand, use of glucomannan for beverages is difficult because of the high viscosity. "Mannan oligosaccharide" is such a compound (or a collection of compounds) that the molecular weight and molecular structure vary with the origin even in the case that constitutional units are common, and thus the characteristics also vary. The "mannan oligosaccharide" described in relation to the invention of Patent Literature 1 is "mannan oligosaccharide such that the number of constitutional monosaccharide units is typically 2 to 90" (in terms of molecular weight, about 360 to 16200). It is not clear from the description of Patent Literature 1, in particular, from data shown in Examples whether agents for accelerating proliferation of Bacteroides containing mannan oligosaccharide or another component are capable not only of promoting the proliferation of Bacteroides but also of largely improving (reducing) the FB ratio.

An object of the present invention is to provide an agent having an effect of reducing the FB ratio in the intestines, that is, an intestinal FB ratio reducing agent, the agent being suitable for use in food/beverage products.

### Solution to Problem

The present inventors found that α-mannan extracted from a yeast (herein, referred to as "yeast mannan" or "yeast α-mannan") has a superior effect of reducing an FB ratio defined as [(occupancy of bacteria belonging to phylum Firmicutes)/(occupancy of bacteria belonging to phylum Bacteroidetes)] (herein, called "an FB ratio in the broad sense") in the intestines. Yeast mannan has higher molecular weight (50000 to 100000 or higher in typical cases) than mannan oligosaccharide as described in Patent Literature 1, but is water-soluble and the viscosity of aqueous solution of yeast mannan is very low. Hence, yeast mannan serves as an intestinal FB ratio reducing agent suitable for use in food/beverage products. As demonstrated later in Examples, use of yeast mannan successfully reduces the FB ratio in a significant manner, as compared with common soluble dietary fibers such as indigestible dextrin.

Focusing on specific bacteria among those belonging to the phylum Firmicutes and those belonging to the phylum Bacteroidetes, the present inventors further found that yeast mannan also has a superior reducing effect on an FB ratio defined as [(total occupancy of bacteria belonging to class Clostridia and bacteria belonging to class Erysipelotrichia among bacteria belonging to phylum Firmicutes)/(occupancy of bacteria belonging to class Bacteroidia among bacteria belonging to phylum Bacteroidetes)] (herein, called "an FB ratio in the narrow sense").

The scope of the present invention encompasses the following items.
[Item 1] An FB ratio reducing agent containing yeast mannan, wherein an FB ratio is defined as [(occupancy of bacteria belonging to phylum Firmicutes)/(occupancy of bacteria belonging to phylum Bacteroidetes)] in the intestines.
[Item 2] An FB ratio reducing agent containing yeast mannan, wherein an FB ratio is defined as [(total occupancy of bacteria belonging to class Clostridia and bacteria belonging to class Erysipelotrichia among bacteria belonging to phylum Firmicutes)/(occupancy of bacteria belonging to class Bacteroidia among bacteria belonging to phylum Bacteroidetes)] in the intestines.
[Item 3] The FB ratio reducing agent according to item 1 or 2, wherein Bacteroides thetaiotaomicron is included in bacteria belonging to the phylum Bacteroidetes or bacteria belonging to the class Bacteroidia among bacteria belonging to the phylum Bacteroidetes.
[Item 4] A food product composition for FB ratio reduction, the food product composition containing the FB ratio reducing agent according to any one of items 1 to 3.

### Advantageous Effects of Invention

Use of the FB ratio reducing agent of the present invention, the agent containing yeast mannan, enables production of food product compositions for FB ratio reduction that have a superior FB ratio reducing effect and allow easy ingestion.

### Brief Description of Drawings

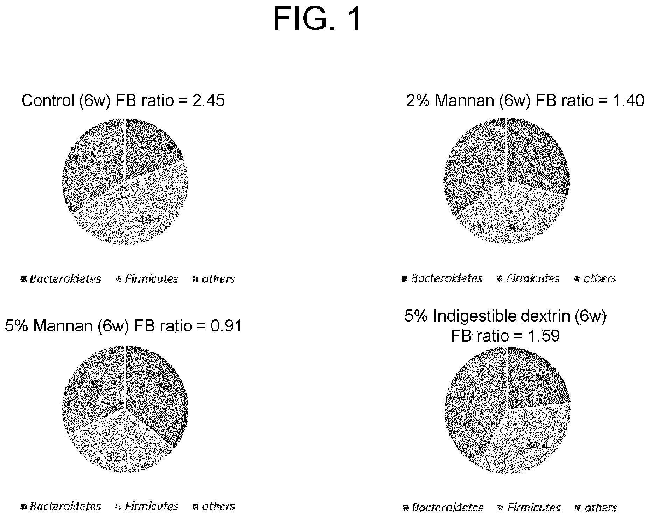

[Figure 1] Figure 1 shows pie charts corresponding to Table 1 (FB ratios in the broad sense) in Experiment 1 (normal diet) in Example 1 and representing fractions (occupancies) of bacteria. "Control" corresponds to "Comparative Example 1" (5% cellulose), "2% Mannan" and "5% Mannan" correspond to "Example 1" and "Example 2", respectively, and "5% Indigestible dextrin" corresponds to "Comparative Example 2". Some numerical values in the pie charts are slightly different from the corresponding numerical values in Table 1 because of rounding.

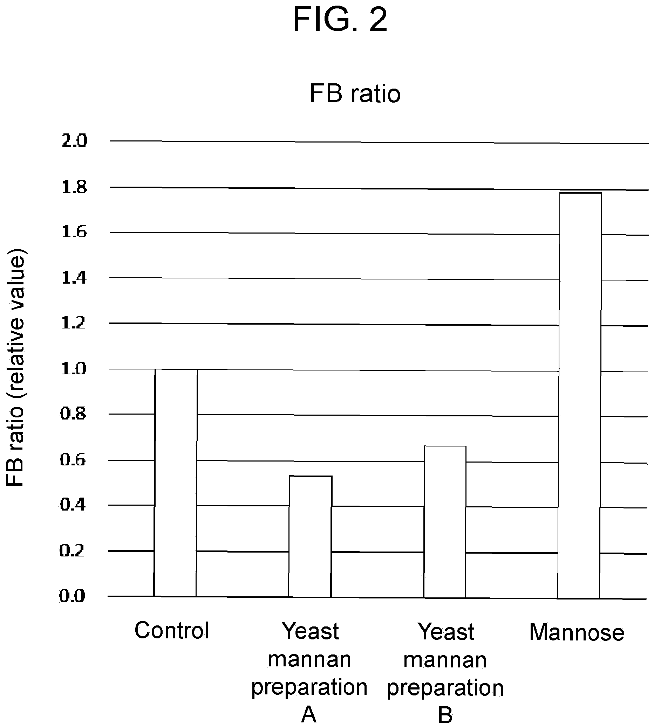

[Figure 2] Figure 2 shows a bar graph corresponding to Table 5 in Example 5 and representing mean values of FB ratios for test subjects.

### Description of Embodiments

### [FB Ratio Reducing Agent]

The FB ratio reducing agent of the present invention contains yeast mannan, and may contain only yeast mannan (consist of yeast mannan) as an active ingredient for the purpose of FB ratio reduction, or contain another active ingredient for the same purpose in addition to yeast mannan. The concept of this "FB ratio" encompasses both "an FB ratio in the broad sense" and "an FB ratio in the narrow sense" described herein.

### (1) Yeast Mannan

In the present invention, yeast mannan refers to a substance containing at least one of a yeast-derived "α-mannan" and a yeast-derived "α-mannan-protein complex". Both of the α-mannan and the α-mannan-protein complex are main components constituting yeast cell walls.

The above-mentioned "α-mannan" is a polysaccharide formed through polymerization of D-mannose via an α1,6-bond, an α1,2-bond, or an α1,3-bond, and a side chain formed of one or two or more mannopyranose moieties may be bonding to the main chain via an α1,2-bond or an α1,3-bond. This α-mannan is water-soluble.

The above-mentioned "α-mannan-protein complex" is a water-soluble glycoprotein in which one or two or more α-mannan chains are bonding to a protein locally present in yeast cell walls via an N-glycoside bond or an O-glycoside bond. As with the case with the above "α-mannan", each of the "α-mannan chains" bonding to the glycoprotein is a polysaccharide chain formed through polymerization of D-mannose via an α1,6-bond, an α1,2-bond, or an α1,3-bond, and a side chain formed of one or two or more mannopyranose moieties may be bonding to the main chain via an α1,2-bond or an α1,3-bond.

Yeast mannan may be collected in any manner as long as being derived from yeast. For example, yeast mannan formed by damaging yeast cell walls with an enzyme, chemical treatment, or the like may be separated and collected (extracted), and yeast mannan discharged by a yeast into a medium may be collected.

Yeast mannan is readily available as a commercially available product. Specific examples thereof include mannan from Saccharomyces cerevisiae M7504 (Sigma-Aldrich Japan). Alternatively, yeast mannan can be prepared from a yeast by using any known method as described in the following.

### (2) Method for Preparing Yeast Mannan

Any known method can be used for preparing yeast mannan. For example, yeast mannan can be obtained through a step of preparing cell walls from a yeast by extracting yeast extract from a yeast used in production of any of beer, sake, bread, and so on to afford residual yeast cell walls, and a step of preparing yeast mannan from the residual yeast cell walls. Yeast cell walls sold under the product name "Kobo Saibo Heki (Yeast Cell Walls in English)" by Asahi Group Foods, Ltd. and other similar commercially available products may be used as a raw material of yeast mannan.

An example of the step of preparing yeast mannan from yeast cell walls is a step including a step of obtaining a crude extract of yeast mannan from yeast cell walls (herein, called the "crude extraction step") and, as necessary, an additional subsequent step of purifying the crude extract of yeast mannan to obtain a purified product of yeast mannan (herein, called the "purification step"). In some cases, a step of preparing cell walls from a yeast is performed before the crude extraction step; however, such a step is unnecessary in the case that yeast cell walls produced in advance are used as a raw material. In the crude extraction step, for example, methods of hot water (including dilute alkalis) extraction (e.g., see JP 1989(S64)-3479 B and JP 1983(S58)-109423 A), autodigestion (e.g., see JP 1983(S58)-57153 B), and enzymatic digestion with a cell-wall-digesting enzyme (e.g., see JP 1983(S59)-40126 B) can be used. In the purification step, for example, protein removal treatment with hydrochloric acid or the like, alcohol precipitation treatment, protease treatment, and treatment with chromatography can be performed.

Herein, crude extracts of yeast mannan and purified products of yeast mannan are collectively called "yeast mannan preparations". The above-described commercially available yeast mannan products are also included in the category of yeast mannan preparations. Various contaminants present in yeast cell walls or generated through treatment for preparation of yeast mannan (e.g., glucan, protein, mannose) may be contained in addition to yeast-derived α-mannan and/or an α-mannan-protein complex in yeast mannan preparations. The yeast mannan content of a yeast mannan preparation is not limited to particular values, and may be any content such that the yeast mannan contains such a content of yeast α-mannan, described later, that the operative advantageous effects of the present invention are exerted when the yeast mannan preparation is used as yeast mannan. Which method is used in the crude extraction step, whether the purification step is performed, which treatment is used if the purification step is performed, and so on may be appropriately determined so that a yeast mannan preparation containing a desired content of yeast α-mannan can be obtained. Those skilled in the art could prepare yeast mannan containing such a content of yeast α-mannan that the operative advantageous effects of the present invention are exerted without problem in accordance with a common method and conditions. In other words, yeast mannan prepared by those skilled in the art in accordance with a common method and conditions contains such a content of yeast α-mannan that the operative advantageous effects of the present invention are exerted without problem.

The yeast to be used as a raw material in the above method for preparing yeast mannan is not limited to a particular yeast, and may be any yeast whose cell walls contain the above-described α-mannan and the above-described α-mannan-protein complex as constituent components. The yeast may be any yeast of Saccharomyces spp., Shizosaccharomyces spp., Pichia spp., Candida spp., Kluyveromyces spp., Williopsis spp., Debaryomyces spp., Galactomyces spp., Torulaspora spp., Rhodotorula spp., Yarrowia spp., Zygosaccharomyces spp., and so on, and is preferably Saccharomyces sp. Those skilled in the art could culture a yeast that does not cause any problem in using the yeast as a raw material for preparing yeast mannan that exerts the operative advantageous effects of the present invention in accordance with a common method and conditions. In other words, a yeast cultured by those skilled in the art in accordance with a common method and conditions can be used as a raw material for preparing yeast mannan that exerts the operative advantageous effects of the present invention without problem.

The above-described yeast to be used as a raw material of yeast mannan is preferably an edible yeast, and more preferably a yeast used as a bread yeast or a beer yeast. Specific examples thereof include Saccharomyces cerevisiae, Schizosaccharomyces pombe, Saccharomyces pastorianus, Saccharomyces paradoxus, Saccharomyces mikatae, Saccharomyces bayanus, Saccharomyces kudriavzevii, Kluyveromyces lactis, Candida utilis, Candida albicans, Candida tropicalis, Candida lypolitica, and Candida sake. The above-described yeast to be used as a raw material of yeast mannan is preferably Saccharomyces cerevisiae, Saccharomyces pastorianus, or Saccharomyces bayanus.

### (3) Method for Calculating Content Ratio of Yeast α-Mannan

The yeast mannan preparation obtained with the above preparation method may contain a yeast-derived α-mannan-protein complex (i.e., a protein moiety bonding to an α-mannan chain). Herein, yeast-derived α-mannan and the α-mannan chain part of a yeast-derived α-mannan-protein complex are collectively referred to as "yeast α-mannan" ("yeast α-mannan" can be said to be pure yeast mannan). The content ratio of yeast α-mannan in yeast mannan somewhat varies among methods for preparing yeast mannan, but is in a generally constant range, typically, 30 to 95%. The content ratio of yeast α-mannan is not limited to particular values, and may be any value such that the operative advantageous effects of the present invention are exerted in using a yeast mannan preparation as yeast mannan. Those skilled in the art could prepare yeast mannan with such a content ratio of yeast α-mannan that the operative advantageous effects of the present invention are exerted without problem in accordance with a common method and conditions. In other words, yeast mannan prepared by those skilled in the art in accordance with a common method and conditions has such a content ratio of yeast α-mannan that the operative advantageous effects of the present invention are exerted without problem.

The content ratio of yeast α-mannan in a yeast mannan preparation is calculated in the following manner. First, mannose in sample (A) of a yeast mannan preparation and mannose in sample (B) of a hydrolysis product of the yeast mannan preparation are quantified. Here, sample (B) is a specimen obtained by treating sample (A) with sulfuric acid to hydrolyze all the glycans in sample (A) into monosaccharides and then neutralizing the resultant. Mannose in each specimen can be quantified, for example, by using high-performance liquid chromatography (HPLC), and may be quantified by using a method performed in the general incorporated foundation Japan Food Research Laboratories. Subsequently, the amount of mannose in sample (A) is subtracted from the amount of mannose in sample (B), and the resulting value is multiplied by 0.9 to exclude the amount of water bonded through hydrolysis of yeast α-mannan, giving a calculated value of the content of yeast α-mannan in the yeast mannan preparation. Finally, the ratio of the content of yeast α-mannan to the weight of sample (A) is calculated, thus determining the ratio of yeast α-mannan in the yeast mannan preparation, that is, the content ratio of yeast α-mannan.

It is not needed to use a yeast mannan preparation with the content ratio of yeast α-mannan being 100% for the agents of the present invention, and any yeast mannan preparation with the content ratio of yeast α-mannan being in the above common range may be used.

The dosage form of the FB ratio reducing agent of the present invention is not limited to a particular dosage form, and may be a powder or a solution (dispersion) using water or other solvent. The FB ratio reducing agent of the present invention is typically blended in a food product composition for FB ratio reduction for use, and hence is preferably in a dosage form suitable for production of such food product compositions.

In the present invention, an "FB ratio in the broad sense" is defined as [(occupancy of bacteria belonging to phylum Firmicutes)/(occupancy of bacteria belonging to phylum Bacteroidetes)], and an "FB ratio in the narrow sense" is defined as [(total occupancy of bacteria belonging to class Clostridia and bacteria belonging to class Erysipelotrichia among bacteria belonging to phylum Firmicutes)/(occupancy of bacteria belonging to class Bacteroidia among bacteria belonging to phylum Bacteroidetes)].

The phylum Bacteroidetes includes the class Bacteroidia, the class Flavobacteriia, and the class Sphingobacteriia.

The class Bacteroidia includes the genus Bacteroides, the genus Parabacteroides, the genus Prevotella, the genus Paraprevotella, the genus Alistipes, the genus Barnesiella, the genus Butyricimonas, and the genus Odoribacter.

The class Flavobacteriia includes the genus Flavobacterium. The class Sphingobacteriia includes the genus Sphingobacterium.

In intestinal microflora, bacteria belonging to the class Flavobacteriia and bacteria belonging to the class Sphingobacteriia are detected but the occupancy are very low. Therefore, "occupancy of bacteria belonging to phylum Bacteroidetes" may be substantially regarded as "occupancy of bacteria belonging to class Bacteroidia" in calculating FB ratios. In the present invention, it is preferable that Bacteroides thetaiotaomicron be included in "bacteria belonging to phylum Bacteroidetes" or "bacteria belonging to class Bacteroidia among bacteria belonging to phylum Bacteroidetes" in calculating FB ratios. In addition to Bacteroides thetaiotaomicron, Bacteroides ovatus and so on are included in bacteria belonging to the class Bacteroidia.

The phylum Firmicutes include the class Clostridia, the class Erysipelotrichia, and the class Bacilli.

The class Clostridia includes the genus Clostridium, the genus Blautia, SMB53, the genus Coprococcus, the genus Dorea, the genus Lachnospira, the genus Roseburia, the genus Ruminococcus, and the genus Veillonella.

The class Erysipelotrichia includes the genus Allobaculum, the genus Catenibacterium, the genus Coprobacillus, and the genus Holdemania.

The class Bacilli includes the genus Bacillus, the genus Lactobacillus, the genus Pediococcus, the genus Lactococcus, the genus Leuconostoc, and the genus Weissella.

To calculate an FB ratio in the narrow sense, a total occupancy of bacteria belonging to the class Clostridia and bacteria belonging to the class Erysipelotrichia among bacteria belonging to the phylum Firmicutes is used. This is for the purpose of excluding the class Bacilli among bacteria belonging to the phylum Firmicutes, because the class Bacilli includes lactic acid bacteria and Bacillus subtilis, which are recognized as good bacteria in most common cases.

The FB ratio reducing agent of the present invention, or yeast mannan contained as an active ingredient of it can reduce the FB ratio in the intestines of any of humans, mice, and other mammals through being ingested. The FB ratio varies among test conditions such as feed conditions (intake, and a normal diet or a high-fat diet) in a test with experimental animals, and it is difficult to rigidly specify the effect with a numerical range or the like. However, it is suitable that the FB ratio after ingestion of the FB ratio reducing agent be at least reduced from the FB ratio before ingestion under test conditions appropriately set to allow comparison. Alternatively, it is suitable that the FB ratio after ingestion of the FB ratio reducing agent be smaller than that for a control group (a group without ingestion of the FB ratio reducing agent, or a group with ingestion of cellulose), preferably smaller than that for a group with additional ingestion of another component such as "indigestible dextrin". In one aspect of the present invention, the FB ratio reducing agent of the present invention can reduce the numerical value of the FB ratio preferably to 85% or lower, more preferably to 70% or lower, even more preferably to 60% or lower (in terms of the amount of reduction, 15% or more, 30% or more, and 40% or more, respectively) of that for a control group (a group without ingestion of the FB ratio reducing agent, or a group with ingestion of cellulose). Here, there is no limitation to the reduction, but, if necessary, the reduction may be set, for example, to a reduction preferably of up to 10%, more preferably of up to 15%, even more preferably of up to 20% (in terms of the amount of reduction, up to 90%, up to 85%, and up to 80%, respectively).

The occupancy of bacteria belonging to each class or genus to calculate an "FB ratio in the broad sense" and "FB ratio in the narrow sense" can be determined by using any technique commonly used for analysis of intestinal microflora. For example, an occupancy of desired bacteria can be determined in the following manner: a DNA is prepared from a fecal sample; a region of ribosomal RNA used in phylogenetic analysis of microorganisms (typically, 16S rRNA) is amplified to acquire a huge number of nucleotide sequences by using a next-generation sequencer; and homology search for a database and phylogenic systematic analysis are performed.

### [Food Product Composition]

The food product composition for FB ratio reduction of the present invention contains the FB ratio reducing agent of the present invention and an additional component compatible with the form of the food product composition. It should be noted that the scope of the food product composition of the present invention does not encompass natural materials containing yeast mannan, which is the active ingredient of the FB ratio reducing agent, in the form of a yeast with cell walls but produced not as a composition, and cooked or processed products thereof, such as fermented food products themselves, in which a yeast remains.

The form of the food product composition is not limited to a particular form, and the food product composition may be in any form of known food/beverage products in which the FB ratio reducing agent of the present invention can be blended. Examples of the form of the food product composition include supplements (dietary supplements) in tablets, capsules, powders (granules), and other dosage forms; soft drinks (e.g., carbonated drinks, non-alcoholic drinks, juices, coffee drinks, tea drinks, mineral waters, sports drinks), milk drinks, soymilk drinks, fermented milks, lactic acid bacteria drinks, cocoas, alcoholic drinks (e.g., beer, low-malt beer, other brewed alcoholic drinks, liqueur, Japanese sake, amazake, wine, shochu), drinks prepared from instant powders, and other drinks; bread, cereal, confectionery (e.g., biscuits, cookies, chocolates), processed products (e.g., katsuobushi, shiokara, kusaya), salted vegetables (e.g., nukadzuke, kimchi, pickles), fermented food products (e.g., natto, cheese, yogurt), seasonings (e.g., soy sauce, miso, vinegar), food additives, and other food products.

The content of the FB ratio reducing agent in the food product composition or the content of yeast mannan as the active ingredient are not limited to particular values, and each may be any value such that the FB ratio reducing effect of the present invention is exerted. The contents may be appropriately set in view of the form of and production method for the food product composition, the amount of the active ingredient per meal or day, as necessary, and so on. In using a yeast mannan preparation as the FB ratio reducing agent of the present invention, the content of yeast α-mannan as an active ingredient can be controlled within such a range that the operative advantageous effects of the present invention are exerted, via the weight of the yeast mannan preparation and the ratio of yeast α-mannan contained in the yeast mannan preparation (i.e., the content ratio of yeast α-mannan).

In the case that the food product composition of the present invention is a supplement, for example, the content of the FB ratio reducing agent can be such that the content of yeast α-mannan contained therein is typically 5% or more, preferably 10% or more, and typically 75% or less, to the total mass of the food product composition (supplement). In the case that the food product composition of the present invention is a beverage, the content of the FB ratio reducing agent can be such that the content of yeast α-mannan contained therein is typically 0.04% or more, preferably 0.1% or more, and typically 7.5% or less, preferably 4% or less, to the total mass of the food product composition (beverage).

Raw materials or components of the food product composition of the present invention other than the FB ratio reducing agent may be the same as those of the corresponding conventional food product compositions.

For example, the food product composition of the present invention in the form of a supplement may contain, in addition to the FB ratio reducing agent, at least one selected from components contained in common or known supplements, such as excipients, sweeteners, coloring agents, flavors, and other components.

The food product composition of the present invention in the form of a beverage may contain, in addition to the FB ratio reducing agent, at least one selected from components contained in common or known beverages, such as water (carbonated water), coffee extract, tea leaf extract, fruit juice, other extract of plant raw material, milk, soymilk, fermented milk, sweeteners, acidulants, coloring agents, and flavors.

The food product composition of the present invention as described can be produced in the same manner as the corresponding conventional food product compositions are produced, except that a step of adding as a component the FB ratio reducing agent of the present invention is further included.

The food product composition may be produced, for example, as a food with health claims (i.e., a food with nutrient function claims, a food for specified health uses, or a food with functional claims), or a therapeutic diet (i.e., a diet for treatment, or a diet prepared on the basis of a menu made by a nutritionist or the like with reference to a dietary slip from a physician), a diet for dietary therapy, or a care diet.

The standard intake of the food product composition (the amount of the active ingredient) per meal or per day may be appropriately set, for example, according to the age, body weight, and sex of a subject whose FB ratio is to be reduced, the FB ratio before ingestion, and other indexes for the subject, or on the basis of non-clinical or clinical test results or the like. The period to ingest the food product composition may be appropriately set, and it is preferred to ingest repeatedly on a daily basis for a long period of time. In one aspect of the present invention, the food product composition of the present invention is ingested so that the intake of yeast α-mannan contained therein as an active ingredient is preferably 0.1 g/day or more, more preferably 0.2 g/day or more, even more preferably 0.3 g/day or more, and, preferably 4 g/day or less, more preferably 2.5 g/day or less, even more preferably 1.5 g/day or less.

The use of the food product composition of the present invention, "FB ratio reduction", may be displayed directly or indirectly. Examples of direct display include description printed on a tangible object such as a product itself, a package, a container, a label, and a tag, and examples of indirect display include publicity and advertisement in places or by means, for example, on a website, in a store, in an exhibition, with a billboard, with a bulletin board, in a newspaper, in a magazine, on TV, on radio, with a mail, or with an e-mail. In producing the food product composition of the present invention as a food with functional claims, for example, functions based on the operative advantageous effects of yeast mannan contained as a functional substance may be displayed, for example, as "reduce (improve) the FB ratio".

### [Another Aspect of Present Invention]

The present invention provides, as one aspect, a method for reducing an FB ratio (in the broad sense) defined as [(occupancy of bacteria belonging to phylum Firmicutes)/(occupancy of bacteria belonging to phylum Bacteroidetes)] in the intestines of a human or a non-human mammal (e.g., an experimental animal such as a mouse, or a pet), the method containing allowing the human or non-human mammal to ingest an effective amount of yeast mannan. The present invention provides, as one aspect, a method for reducing an FB ratio (in the narrow sense) defined as [(total occupancy of bacteria belonging to class Clostridia and bacteria belonging to class Erysipelotrichia among bacteria belonging to phylum Firmicutes)/(occupancy of bacteria belonging to class Bacteroidia among bacteria belonging to phylum Bacteroidetes)] in the intestines of a human or a non-human mammal (e.g., an experimental animal such as a mouse, or a pet), the method containing allowing the human or non-human mammal to ingest an effective amount of yeast mannan. Those skilled in the art could divert the inventions of "FB ratio reducing agent" and "food product composition for FB ratio reduction" described above to those inventions of "methods for reducing an FB ratio (in the broad sense or narrow sense)", and the matters on the "FB ratio reducing agent" and "food product composition for FB ratio reduction" described herein can be applied in the same manner.

The present invention provides, as one aspect, use of yeast mannan in production of a food product for reducing an FB ratio (in the broad sense) defined as [(occupancy of bacteria belonging to phylum Firmicutes)/(occupancy of bacteria belonging to phylum Bacteroidetes)] in the intestines of a human or a non-human mammal (e.g., an experimental animal such as a mouse, or a pet). The present invention provides, as one aspect, use of yeast mannan in production of a food product for reducing an FB ratio (in the narrow sense) defined as [(total occupancy of bacteria belonging to class Clostridia and bacteria belonging to class Erysipelotrichia among bacteria belonging to phylum Firmicutes)/(occupancy of bacteria belonging to class Bacteroidia among bacteria belonging to phylum Bacteroidetes)] in the intestines of a human or a non-human mammal (e.g., an experimental animal such as a mouse, or a pet). Those skilled in the art could divert the inventions of "FB ratio reducing agent" and "food product composition for FB ratio reduction" described above to such "use of yeast mannan", and the matters on the "FB ratio reducing agent" and "food product composition for FB ratio reduction" described herein can be applied in the same manner.

### Examples

### (Method for Calculating Content Ratio of Yeast α-Mannan)

In Examples, mannose in sample (SA) of each of yeast mannan preparations X, A, and B obtained by a manner described later, and mannose in sample (SB) of a hydrolysis product of each of yeast mannan preparations X, A, and B were quantified through outsourcing to the general incorporated foundation Japan Food Research Laboratories. On the basis of the quantification results, the content ratio of yeast α-mannan in each of yeast mannan preparations X, A, and B was calculated by using the above-described calculation method.

The specific procedure of the quantification method for mannose in sample (SA) and sample (SB) by the general incorporated foundation Japan Food Research Laboratories was as follows. From each of sample (SA) and sample (SB), a portion of 0.6 g was taken, and each portion was added to sulfuric acid with a concentration of 72%, and the resultant was stirred at room temperature for 1 hour. The sulfuric acid concentration of each specimen solution was diluted to 4% by dilution, and the specimen solutions were then heated in an autoclave (121°C) for 1 hour. The specimen solutions were cooled to room temperature and then neutralized, and water was added to each specimen solution to adjust the volume to 200 mL. The specimen solutions after volume adjustment were each filtered through a filter paper, and the filtrates were each 50-fold diluted with water, and the diluted solutions were each filtrated through a membrane filter. The filtrates were analyzed by high-performance liquid chromatography under the following operation conditions to measure the mannose concentration of each filtrate, and mannose in sample (SA) and sample (SB) was quantified.
Apparatus: LC-20AD (Shimadzu Corporation)
Detector: fluorescence spectrophotometer RF-20Axs (Shimadzu Corporation)
Column: TSKgel SUGAR AXI, φ4.6 mm × 150 mm (Tosoh Corporation)
Column temperature: 60°C
Mobile phase: 0.5 mol/L borate buffer (pH 8.7)
Flow rate: 0.4 mL/min
Injection volume: 20 µL
Fluorescence excitation wavelength: 320 nm
Fluorescence measurement wavelength: 430 nm
Post-column:
   reaction solution: 1 w/v% L-arginine solution;
   flow rate of reaction solution: 0.7 mL/min;
   reaction temperature: 150°C

### [Examples 1 to 4 and Comparative Examples 1 to 3]

### (Yeast Mannan Preparation X)

Ten kilograms of commercially available yeast cell walls (product name: "Kobo Saibo Heki (Yeast Cell Walls in English)", Asahi Group Foods, Ltd.) was suspended in distilled water, and sodium hydroxide was added to the suspension to adjust the pH to 11.0, and the suspension was then boiled at 90°C for 12 hours. The supernatant was collected through centrifugation, and hydrochloric acid was added thereto to adjust the pH to 5.5. A freeze-drying operation provided 2 kg of yeast mannan preparation X. By using the above-described method, the content ratio of yeast α-mannan was calculated to be 50%.

"Yeast mannan (2%)" and "Yeast mannan (5%)" in Examples 1 to 4 indicate that yeast mannan preparation X was mixed with a feed ("AIN93G", produced by Oriental Yeast Co., Ltd.) so that the mass of yeast mannan preparation X was 2% of the mass of the feed in the former case, and 5% of the mass of the feed in the latter case.

### (Cellulose and Indigestible Dextrin)

Cellulose ("CEOLUS UF-F702" produced by Asahi Kasei Chemicals Corporation) was used in Comparative Example 1. Indigestible dextrin ("Fibersol-2" produced by Matsutani Chemical Industry Co., Ltd.) was used in Comparative Example 2.

"Cellulose (5%)" and "indigestible dextrin (5%)" in Comparative Examples 1 and 2 indicate the weight or mass ratios of cellulose and indigestible dextrin, respectively, mixed in a feed ("AIN93G", Oriental Yeast Co., Ltd.) to the feed.

### (Experiment 1: Normal Diet)

As described above, yeast mannan preparation X, cellulose, and indigestible dextrin were each mixed with a feed ("AIN93G", Oriental Yeast Co., Ltd.) to prepare test feeds. Four-week-old male ICR mice were grouped so that each group consisted of nine mice, and allowed to freely ingest each test feed for 6 weeks. A library was prepared in accordance with a protocol provided by Illumina, Inc. (Document 1 shown below) by using DNAs extracted from mouse feces, as templates, and a forward primer (SEQ ID NO: 1) and a reverse primer (SEQ ID NO: 2) having nucleotide sequences shown below, and sequencing was performed for a V4 region of 16S rDNA (a DNA encoding 16S rRNA) by using an MiSeq Reagent Kit version 2 (300 cycles) (Illumina, Inc.) and an MiSeq. The sequences acquired were analyzed by using QIIME v.1.8.0 (Document 2 shown below), R, and SPSS to calculate FB ratios in the broad sense and FB ratios in the narrow sense.

SEQ ID NO: 1: 5'-TCGTCGGCAGCGTCAGATGTGTATAAGAGACAGGTGCCAG
CMGCCGCGGTAA-3'

SEQ ID NO: 2: 5'-GTCTCGTGGGCTCGGAGATGTGTATAAGAGACAGGACTAC

HVGGGTATCTAATCC-3'

Document 1: 16S Metagenomic Sequencing Library Preparation.(http://support.illumina.com/documents/documentat ion/chemistry_documentation/16s/16s-metagenomic-library-prep-guide-15044223-b.pdf).

Document 2: QIIME allows analysis of high-throughput community sequencing data. Caporaso JG, Kuczynski J, Stombaugh J, et al. Nat Methods (2010) 7, 335.

No significant difference was found for food consumption and body weight during the test period over 6 weeks. No significant intergroup difference was found for the total number of intestinal bacterial cells (approximately 1011 copies/g feces) at the end of the test period (not shown).

Table 1 shows FB ratios in the broad sense after ingestion of different test feeds for 6 weeks and fractions (percentages as the total number of bacterial cells was assumed as 100%) of intestinal bacteria involved in the calculation. Figure 1 shows pie charts corresponding to the table and representing fractions of the phylum Bacteroidetes, the phylum Firmicutes, and other intestinal bacteria.

### [Table 1]

**Table 1. FB ratios after ingestion of different materials for 6 weeks**

| | Material | FB ratio | Fraction (%) of phylum Bacteroidetes | Fraction (%) of phylum Firmicutes |
|---|---|---|---|---|
| Example 1 | Yeast mannan (2%) | 1.40 | 29.0±4.3 | 36.4±9.4 |
| Example 2 | Yeast mannan (5%) | 0.91 | 35.8±0.8 | 32.4±9.0 |
| Comparative Example 1 | Cellulose (5%) | 2.45 | 19.7±2.3 | 46.5±1.9 |
| Comparative Example 2 | Indigestible dextrin (5%) | 1.59 | 23.1±2.8 | 34.4±4.1 |

Table 2 shows FB ratios in the narrow sense after ingestion of different test feeds for 6 weeks and fractions (percentages as the total number of bacterial cells was assumed as 100%) of intestinal bacteria involved in the calculation.

### [Table 2]

**Table 2. FB ratios in narrow sense after ingestion of different materials for 6 weeks**

| | Material | FB ratio in narrow sense | Fraction (%) of class Bacteroidia | Fraction (%) of class Clostridia and class Erysipelotrichia |
|---|---|---|---|---|
| Example 1 | Yeast mannan (2%) | 0.63 | 29.0±4.3 | 18.2±10.1 |
| Example 2 | Yeast mannan(5%) | 0.56 | 35.7±0.8 | 20.1±8.3 |
| Comparative Example 1 | Cellulose (5%) | 1.26 | 19.7±2.3 | 24.8±3.1 |
| Comparative Example 2 | Indigestible dextrin (5%) | 1.21 | 23.1±2.8 | 27.5±4.6 |

### (Experiment 2: High-Fat Diet)

Six-week-old male C57BL/6J mice were grouped so that each group consisted of 10 mice, and yeast mannan preparation X was orally administered (5 days/week) at a dose of 300 or 1000 mg/kg/day for 6 weeks while the mice were grown with the high-fat diet HFD (produced by Oriental Yeast Co., Ltd.). Analysis of 16S rDNA was performed with the same procedure as in Experiment 1 to calculate FB ratios in the broad sense and FB ratios in the narrow sense.

Table 3 shows FB ratios in the broad sense after ingestion of yeast mannan preparation X for 6 weeks and fractions (percentages as the total number of bacterial cells was assumed as 100%) of intestinal bacteria involved in the calculation.

### [Table 3]

**Table 3. FB ratios after administration of yeast mannan for 6 weeks (conditions with high-fat diet)**

| | Material (dose) | FB ratio | Fraction (%) of phylum Bacteroidetes | Fraction (%) of phylum Firmicutes |
|---|---|---|---|---|
| (Example 3) | Yeast mannan (300mg/kg/day) | 2.78 | 25.4±2.2 | 70.6±3.0 |
| (Example 4) | Yeast mannan (1000mg/kg/day) | 2.00 | 32.3±4.0 | 64.7±3.9 |
| (Comparative Example 3) | No administration | 3.54 | 21.2±1.9 | 75.1±1.8 |

Table 4 shows FB ratios in the narrow sense after ingestion of yeast mannan preparation X for 6 weeks and fractions (percentages as the total number of bacterial cells was assumed as 100%) of intestinal bacteria involved in the calculation.

### [Table 4]

**Table 4. FB ratios in narrow sense after administration of yeast mannan for 6 weeks (conditions with high-fat diet)**

| | Material (dose) | FB ratio in narrow sense | Fraction (%) of class Bacteroidia | Fraction (%) of class Clostridia and class Erysipelotrichia |
|---|---|---|---|---|
| Example 3 | Yeast mannan (300mg/kg/day) | 2.29 | 25.4±2.2 | 58.3±6.1 |
| Example 4 | Yeast mannan (1000mg/kg/day) | 1.60 | 32.3±4.0 | 51.6±1.7 |
| Comparative Example 3 | No administration | 2.70 | 21.2±1.9 | 57.2±5.3 |

### [Example 5]

Feces from two healthy male individuals and one healthy female individual were used as samples for test. Each sample was cultured in a medium with a yeast mannan preparation or mannose, and FB ratios in areas with a yeast mannan preparation and an area with mannose were compared with an FB ratio in a control area for each sample. FB ratios in Example 5 correspond to "FB ratios in the broad sense" in the present invention.

### (Yeast Mannan Preparation A)

Ten kilograms of commercially available yeast cell walls (product name: "Kobo Saibo Heki (Yeast Cell Walls in English)", Asahi Group Foods, Ltd.) was suspended in distilled water, and sodium hydroxide was added to the suspension to adjust the pH to 11.0, and the suspension was then boiled at 90°C for 12 hours. The supernatant was collected through centrifugation, and hydrochloric acid was added thereto to adjust the pH to 5.5. A freeze-drying operation provided 2 kg of yeast mannan preparation A. Measurement by using the above-described method found the content ratio of yeast α-mannan to be 47.6%.

### (Yeast Mannan Preparation B)

A commercially available reagent (product name: "Mannan from Saccharomyces cerevisiae", produced by Sigma-Aldrich Co. LLC) was used. Measurement by using the above-described method found the content ratio of yeast α-mannan to be 78.8%.

### (Mannose)

A commercially available reagent (product name: "D(+)-Mannose", produced by Wako Pure Chemical Industries, Ltd.) was used.

### (Culture Method)

Cultivation for each sample with reference to a method by Takagi et al. (Takagi, R et al. A Single-Batch Fermentation System to Simulate Human Colonic Microbiota for High-Throughput Evaluation of Prebiotics. PLoS One 11 (2016)).

Each of the media used for the culture was prepared in the following manner.

Distilled water was added to 59.0 g GAM broth (product code: "05422", NISSUI PHARMACEUTICAL CO., LTD.) in a measuring flask to reach a volume of 800 mL, and this solution was autoclaved to afford a GAM medium base.

Yeast mannan preparation A (content ratio of yeast α-mannan: 47.6%) was used to prepare a 0.210 g/L yeast mannan preparation A solution. Calculation confirms this solution to be a 1.00% yeast α-mannan solution. Yeast mannan preparation B (content ratio of yeast α-mannan: 78.8%) was used to prepare a 0.127 g/L yeast mannan preparation B solution. Calculation confirms this solution to be a 1.00% yeast α-mannan solution. Mannose (content ratio of mannose: 100%) was used to prepare a 10.0 g/L mannose solution (1.00% solution).

The above-described yeast mannan preparation A solution, yeast mannan preparation B solution, and mannose solution were each autoclaved.

A medium with yeast mannan preparation A was prepared by mixing 80.0 mL of the GAM medium base described above and 20 mL of the 0.210 g/L yeast mannan preparation A solution described above. A medium with yeast mannan preparation B was prepared by mixing 80.0 mL of the GAM medium base described above and 20 mL of the 0.127 g/L yeast mannan preparation B solution described above. Final content ratios of yeast α-mannan in the media with yeast mannan preparation A and B are both calculated to be 0.20%. A medium with mannose was prepared by mixing 80.0 mL of the GAM medium base described above and 20 mL of the 10.0 g/L mannose solution. A medium of a control condition was prepared by mixing 80.0 mL of the GAM medium base described above and 20 mL of distilled water.

The above-described media were placed in different jar fermentors in a culture apparatus ("Bio Jr. 8", produced by ABLE Corporation). Human feces collected as a sample by a culture swab (Becton, Dickinson and Company) were suspended in 1 mL of 0.1 M phosphate-buffered saline (pH 6.5), and the suspension was put into each jar fermentors. Cultivation at 37°C for 30 hours with aeration with mixed gas of nitrogen and carbon dioxide (nitrogen:carbon dioxide = 4:1) at 15 mL/min.

### (Calculation of FB Ratios)

DNAs were extracted from each culture collected after the above-described culture, and FB ratios were calculated.

Extraction of DNAs from the cultures was performed in accordance with a method by Takagi et al. (Takagi, R et al. A Single-Batch Fermentation System to Simulate Human Colonic Microbiota for High-Throughput Evaluation of Prebiotics. PLoS One 11 (2016)). To 200 µL of each culture, 500 µL of Tris-EDTA (TE)-saturated phenol, 250 µL of lysis buffer (1 M Tris pH 8.0, 0.5 M EDTA), 50 µL of 10% SDS, and 0.3 g of glass beads (φ0.1 mm) were added, and the resultant was vigorously shaken in the vertical direction by using a FastPrep24 (MP Biomedicals) at a power level of 5.0 for 30 seconds. The supernatant was collected through centrifugation, mixed with phenol/chloroform/isoamyl alcohol, and centrifuged, and DNAs were precipitated from the resulting aqueous layer through isopropanol precipitation, and then washed with 70% ethanol, dried, and dissolved in 100 µL of TE for DNA extraction from the culture. The DNAs obtained were used as template DNAs for quantitative PCR.

Bacteria belonging to the phylum Firmicutes and bacteria belonging to the phylum Bacteroidetes, and eubacteria in the DNAs extracted from each culture were detected by using methods shown below, and the fraction of bacteria belonging to the phylum Firmicutes in eubacteria, that is, the occupancy, was calculated. Similarly, the fraction of bacteria belonging to the phylum Bacteroidetes in eubacteria, that is, the occupancy, was calculated.

Bacteria belonging to the phylum Firmicutes were quantified through PCR with primers specific to bacteria belonging to the phylum Firmicutes (forward: 5'-TGAAACTYAAAGGAATTGACG-3', reverse: 5'-ACCATGCACCACCTGTC-3') in accordance with a method by Gregoris et al. (Tristano Bacchetti De Gregoris et al. Improvement of phylum-and class-specific primers for real-time PCR quantification of bacterial taxa. Journal of Microbiological Methods 86 (2011) 351-356).

Bacteria belonging to the phylum Bacteroidetes were quantified through PCR with primers specific to bacteria belonging to the phylum Bacteroidetes (forward: 5'-CRAACAGGATTAGATACCCT-3', reverse: 5'-GGTAAGGTTCCTCGCGTAT-3') in accordance with the method by Gregoris et al.

SsoAdvanced (TM) Universal SYBR (Registered Trademark) Green Supermix (Bio-Rad Laboratories, Inc.) was used as a reagent for quantitative PCR, and QuantStudio3 Real-Time PCR Systems (Thermo Fisher Scientific) were used for PCR reaction and detection.

In quantitative PCR for bacteria belonging to the phylum Firmicutes and bacteria belonging to the phylum Bacteroidetes, in both cases, reaction at 95°C for 5 seconds was followed by 30 cycles of reaction at 95°C for 15 seconds, at 61.5°C for 15 seconds, and at 72°C for 20 seconds, and subsequent reaction at 72°C for 5 seconds.

Eubacteria were quantified through PCR with primers specific to eubacteria (forward: 5'-CGGTGAATACGTTCCCGG-3', reverse: 5'-TACGGCTACCTTGTTACGACTT-3') in accordance with a method by Furet et al. (Furet JP et al. Comparative assessment of human and farm animal faecal microbiota using real-time quantitative PCR. FEMS Microbiol Ecol. 2009 Jun; 68 (3) :351-62) .

SsoAdvanced (TM) Universal SYBR (Registered Trademark) Green Supermix (Bio-Rad Laboratories, Inc.) was used as a reagent for quantitative PCR, and QuantStudio3 Real-Time PCR Systems (Thermo Fisher Scientific) were used for PCR reaction and detection.

In PCR reaction, reaction at 95°C for 20 seconds, at 56°C for 20 seconds, and at 72°C for 30 seconds was repeated for 40 cycles.

The FB ratios of the culture were each calculated as "FB ratio = (occupancy of bacteria belonging to phylum Firmicutes)/(occupancy of bacteria belonging to phylum Bacteroidetes)". For each test subject, relative values of FB ratios in the conditions adding the yeast mannan preparation or mannose were calculated as the FB ratio in the control condition was assumed as 1. Table 5 shows relative values of FB ratios and mean values thereof for the test subjects.

**[Table 5]**

| FB Ratios (assuming control as 1) | | | | |
|---|---|---|---|---|
| | Test subject A (male) | Test subject B (male) | Test subject C (female) | Mean value |
| Control | 1.00 | 1.00 | 1.00 | 1.00 |
| Yeast mannan preparation A | 0.47 | 0.72 | 0.40 | 0.53 |
| Yeast mannan preparation B | 0.50 | 0.76 | 0.75 | 0.67 |
| Mannose | 1.77 | 1.69 | 1.89 | 1.78 |

## Claims

1. An FB ratio reducing agent comprising yeast mannan, wherein an FB ratio is defined as [(occupancy of bacteria belonging to phylum Firmicutes)/(occupancy of bacteria belonging to phylum Bacteroidetes)] in the intestines.

2. An FB ratio reducing agent comprising yeast mannan, wherein an FB ratio is defined as [(total occupancy of bacteria belonging to class Clostridia and bacteria belonging to class Erysipelotrichia among bacteria belonging to phylum Firmicutes)/(occupancy of bacteria belonging to class Bacteroidia among bacteria belonging to phylum Bacteroidetes)] in the intestines.

3. The FB ratio reducing agent according to claim 1 or 2, wherein Bacteroides thetaiotaomicron is included in bacteria belonging to the phylum Bacteroidetes or bacteria belonging to the class Bacteroidia among bacteria belonging to the phylum Bacteroidetes.

4. A food product composition for FB ratio reduction, the food product composition comprising the FB ratio reducing agent according to any one of claims 1 to 3.
